# EUROPEAN PATENT APPLICATION

(11) **EP 4 684 825 A1**
(43) Date of publication of application: **28.01.2026**
(21) Application number: 25189326.9
(22) Date of filing: 14.07.2025
(51) Int. Cl.: A61N 1/36, A61N 1/04

(54) **ELECTRICAL STIMULATION CIRCUIT, ELECTRICAL STIMULATION METHOD, AND STORAGE MEDIUM**

(30) Priority: 26.07.2024 CN 202411009298
(71) Applicant: Fasikl Incorporated, Dallas, TX 75206 (US); Hangzhou Fasikl Technology Co., Ltd, Hangzhou, Zhejiang 310000 (CN)
(72) Inventor: NGUYEN, Jules Anh Tuan, Dallas, 75206 (US); WANG, Wei, Hangzhou City, 310000 (CN); YE, Bing, Hangzhou City, 310000 (CN); WANG, Baitong, Hangzhou City, 310000 (CN)
(74) Representative: Chung, Hoi Kan

(57) **Abstract**

Disclosed are an electrical stimulation circuit, an electrical stimulation method, an electronic device, and a storage medium, where the electrical stimulation circuit includes: a timer for timing; a pulse width modulator connected to the timer, where a first pulse signal is generated when first trigger time set by the timer is up, and a second pulse signal is generated when second trigger time set by the timer is up; a driving circuit connected to the pulse width modulator, where the first pulse signal outputs a first stimulation current, the second pulse signal outputs a second stimulation current through the driving circuit, and the first stimulation current is opposite to the second stimulation current; and a stimulation electrode connected to the driving circuit, where the stimulation electrode is in contact with a plurality of stimulation positions, and configured for randomly outputting the first stimulation current and the second stimulation current.

## Description

### TECHNICAL FIELD

The present invention relates to the technical field of medical devices, and particularly relates to an electrical stimulation circuit, an electrical stimulation method, and a storage medium.

### BACKGROUND

Essential tremor is a common nervous system disease characterized by an involuntary rhythmic oscillation of various parts of the body (especially hands and arms), which severely impairs the daily activities and living quality of patients. Current therapeutic regimens, including medication and invasive surgeries, usually have limited efficacy or bring significant risks and side effects. Therefore, there is an urgent need to solve the problem of essential tremor through innovative and non-invasive methods.

Current medical devices can stimulate nerves by outputting stimulated current through defibrillators to alleviate essential tremor. However, current defibrillators can only be controlled to turn on/off, and cannot achieve dynamic smooth control or precise control of stimulation signal output, so that user experience is poor. Further, the defibrillators in the prior art perform regular stimulation at fixed locations in a fixed stimulation mode. Due to adaption to the stimulation mode, stimulation to the neural system cannot exert its effects of disease relief and treatment.

### SUMMARY

In an embodiment of the present invention, there are provided an electrical stimulation circuit, an electrical stimulation method, an electronic device, and a storage medium to solve the problems existing in the related art. The present invention provides a technical solution as follows:

In a first aspect, the present invention provides an electrical stimulation circuit, and the circuit includes:
a timer, configured for timing;
a pulse width modulator, connected to the timer, where a first pulse signal is generated when first trigger time set by the timer is up, and a second pulse signal is generated when second trigger time set by the timer is up;
a driving circuit, connected to the pulse width modulator, where the first pulse signal outputs a first stimulation current through the driving circuit, the second pulse signal outputs a second stimulation current through the driving circuit, and the first stimulation current is opposite to the second stimulation current; and
a stimulation electrode, connected to the driving circuit, where the stimulation electrode is in contact with a plurality of stimulation positions, and configured for randomly outputting the first stimulation current and the second stimulation current to any stimulation position.

In an implementation, the circuit further includes:
a peripheral interconnection module, where the timer is connected to the pulse width modulator through the peripheral interconnection module.

In an implementation, the circuit further includes:
an analog-to-digital converter, connected to the timer through the peripheral interconnection module, and configured for acquiring an intermediate voltage of the first stimulation current when first acquisition time set by the timer is up, and acquiring an intermediate voltage of the second stimulation current when second acquisition time set by the timer is up; and the intermediate voltage of the first stimulation current and the intermediate voltage of the second stimulation current are used for fault detection.

In an implementation, the circuit further includes:
a voltage/level converter, configured for performing voltage/level conversion of the first pulse signal and the second pulse signal and outputting to the driving circuit, where an input end thereof is connected to the pulse width modulator, and an output end thereof is connected to the driving circuit.

In an implementation, the driving circuit includes four transistors, the four transistors are combined to form an H-bridge circuit, and a load of the H-bridge circuit is connected to the stimulation electrode.

In an implementation, the H-bridge circuit includes a first n-type metal-oxide-semiconductor (NMOS) transistor, a second NMOS transistor, a first p-type metal-oxide-semiconductor (PMOS) transistor, and a second PMOS transistor, where a source electrode of the first PMOS transistor is connected to a source electrode of the second PMOS transistor and connected to a power supply, a gate electrode of the first PMOS transistor and a gate electrode of the second PMOS transistor are both connected to the same voltage/level converter, and a drain electrode of the first PMOS transistor and a drain electrode of the second PMOS transistor are connected to a positive pole and a negative pole of the stimulation electrode, respectively; the drain electrode of the first PMOS transistor is connected to the drain electrode of the first NMOS transistor, the drain electrode of the second PMOS transistor is connected to the drain electrode of the second NMOS transistor, the gate electrode of the first NMOS transistor and the gate electrode of the second NMOS transistor are connected to another voltage/level converter, and the source electrode of the first NMOS transistor and the source electrode of the second NMOS transistor are connected and grounded through a constant current driver.

In an implementation, the first PMOS transistor and the second PMOS transistor are controlled to be in the "on" state at the first trigger time, so that the stimulation current flows to the stimulation electrodes to neutralize a voltage difference between two stimulation electrodes.

In an implementation, when the first PMOS transistor and the second PMOS transistor are both in the "on" state, the first NMOS transistor is controlled to be in the "on" state, and the stimulation current flows through the first PMOS transistor and the first NMOS transistor to the constant current driver and then a grounding location.

In an implementation, when the first PMOS transistor, the second PMOS transistor, and the first NMOS transistor are all in the "on" state, but the first PMOS transistor is turned off, the stimulation current flows to the stimulation electrode.

In an implementation, the stimulation positions include a corresponding position of a radial nerve, a corresponding position of a median nerve, and a corresponding position of an ulnar nerve.

In an implementation, the following is further included:
a DC/DC boost regulator, where a feedback branch thereof is connected to a digital-to-analog converter, and the digital-to-analog converter is connected to a microcontroller and configured for modifying a power supply voltage according to required output current.

In a second aspect, the present invention provides an electrical stimulation method, and the method is applied to the above electrical stimulation circuit and includes the following steps:
acquiring a stimulation demand, and activating the timer according to the stimulation demand;
generating the first stimulation current when the first trigger time set by the timer is up;
generating the second stimulation current when the second trigger time set by the timer is up; the first stimulation current is opposite to the second stimulation current; and
randomly switching the stimulation positions, so that the stimulation electrode outputs the first stimulation current and the second stimulation current towards the stimulation positions.

In an implementation, a method of generating the first stimulation current and the second stimulation current includes the following steps:
randomly setting pulse parameters based on a preset specified range, where the pulse parameters include a current amplitude, a pulse interval and a pulse width; and
generating the first stimulation current and the second stimulation current based on the pulse parameters.

In an implementation, the method further includes the following steps:
acquiring the intermediate voltage of the first stimulation current and the intermediate voltage of the second stimulation current;
calculating a first-stage stimulation current and a second-stage stimulation current according to the intermediate voltage of the first stimulation current and the intermediate voltage of the second stimulation current, respectively; and
comparing the first-stage stimulation current and the second-stage stimulation current with a preset target current, and generating a fault signal for triggering a fault event when a difference between the first-stage stimulation current and the target current is greater than a fault threshold, and/or the difference between the second-stage stimulation current and the target current is greater than the fault threshold.

**In** an implementation, the method further includes the following steps:
generating an increment signal and sending the increment signal to a counter when a fault event occurs, so that the counter counts up; generating a decrement signal and sending the decrement signal to the counter when no fault event occurs, so that the counter counts down; and
generating a standby instruction when a cumulative count value of the counter exceeds a preset threshold, where the standby instruction is used to control the stimulation electrode to stop outputting the stimulation current.

In an implementation, the method further includes the following steps:
generating a test pulse with a preset amplitude when the standby instruction is executed; and
when determining that an output current of the stimulation electrode matches the test pulse, restoring the stimulation electrode to output the first stimulation current and the second stimulation current.

In an implementation, the method further includes the following steps:
obtaining the required output current and adjusting the power supply voltage of the electrical stimulation circuit according to the required output current.

In a third aspect, in an embodiment of the present invention, there is provided a computer-readable storage medium on which a computer program is stored, and when the computer program is executed on a computer, the method in any one of the implementations in the above aspects is executed.

The advantages or beneficial effects of the above technical solutions at least include:
The present invention makes the stimulation mode become random, and by randomly outputting the corresponding stimulation current to any stimulation position, the present invention prevents the neural system of a user from adapting to the stimulation mode and negating the therapeutic effect; secondly, the present invention generates the first stimulation current and the second stimulation current by means of precise timing, and the first stimulation current and the second stimulation current correspond to the first-stage stimulation and the second-stage stimulation respectively, which can accurately control a dose of charge on a patient's body and ensure a charge balance so as to ensure safety.

The above summary is only for the purpose of illustration, and is not intended to limit the present invention in any way. In addition to the exemplary aspects, implementations and features described above, further aspects, implementations and features of the present invention will become apparent with reference to the accompanying drawings and the detailed description below.

### BRIEF DESCRIPTION OF DRAWINGS

In the accompanying drawings, unless otherwise specified, same reference numerals in a plurality of accompanying drawings indicate the same or similar components or elements. These accompanying drawings are not necessarily drawn to scale. It should be understood that these accompanying drawings only depict some implementations disclosed in the present invention, and should not be construed as limiting the scope of the present invention.
FIG. 1 is a schematic diagram of an electrical stimulation circuit of the present invention.
FIG. 2 is a schematic diagram of three stages of a stimulation pulse in the present invention.
FIG. 3 is a schematic diagram of adjusting an output current of an H-bridge circuit in each stage of the present invention.
FIG. 4 is a schematic diagram of an H-bridge circuit in the "on" state at t1-t2 of the present invention.
FIG.5 is a schematic diagram of an H-bridge circuit in the "on" state at t2-t3 of the present invention.
FIG.6 is a schematic diagram of an H-bridge circuit in the "on" state at t3-t4 of the present invention.
FIG. 7 is a schematic diagram of monitoring a circuit in an actual output current process of the present invention.
FIG. 8 is a flowchart of fault detection in the present invention.
FIG. 9 is a schematic diagram of a circuit connection of a DC/DC boost regulator of the present invention.
FIG. 10 is a schematic diagram of the present invention where a voltage of a high-voltage power supply can increase or decrease in real time according to a value of output current.

### DETAILED DESCRIPTION OF THE EMBODIMENTS

Only some exemplary embodiments are briefly described below. Just as those skilled in the art recognize, the described embodiments can be modified in various ways without departing from the spirit or scope of the present invention. Therefore, the accompanying drawings and the description are considered to be essentially exemplary rather than limiting.

### Embodiment 1

In this embodiment, there is provided an electrical stimulation circuit that can be applied to a wearable medical device. The circuit provides targeted electrical stimulation to a radial nerve, a median nerve and an ulnar nerve located in a wrist region and achieves long-term tremor relief by optimizing the transmission of peripheral nerve stimulation (PNS). Further, the circuit improves the effectiveness and personalization of the treatment of essential tremor, providing a promising solution to patients seeking to improve symptom management level and living quality.

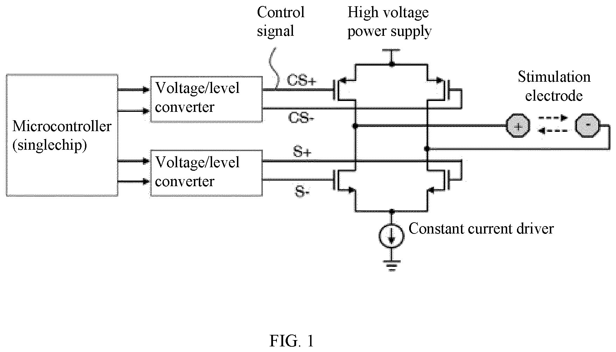

As shown in FIG. 1, the electrical stimulation circuit in this embodiment includes a microcontroller, a voltage/level converter, a driving circuit, and a stimulation electrode. An input/output (I/O) pin of the microcontroller is connected to the voltage/level converter and the driving circuit in succession, a load of the driving circuit is connected to the stimulation electrode, the stimulation electrode is configured for contact with a specified stimulation position in the human body, and the microcontroller controls the logic of transistors in the driving circuit to generate a stimulation current. The stimulation positions include a corresponding position of a radial nerve, a corresponding position of a median nerve, and a corresponding position of an ulnar nerve.

An input end of the voltage/level converter in this embodiment is connected to a pulse width modulator, and an output end thereof is connected to the driving circuit, where the voltage/level converter is configured for converting a low-voltage pulse signal generated by the microcontroller into a signal suitable for driving a gate electrode of the transistor in the driving circuit.

The microcontroller may be internally provided with a pulse width modulator and a timer, where the timer is connected to the pulse width modulator through a peripheral interconnection (PPI) module, and the timer is configured for timing. Once the timer is triggered, a cascade effect will automatically activate all related modules at a predefined and precise timing interval. For example, the pulse width modulator is activated to adjust an amplitude or a frequency of an output signal and control an on/off state of the transistor in the driving circuit connected to the microcontroller, so as to generate a corresponding stimulation current.

**In** this embodiment, the timer is configured for timing, and after the timer is turned on, counting starts. When first trigger time set by the timer is up, the pulse width modulator is triggered to generate a first pulse signal, and when second trigger time set by the timer is up, the pulse width modulator is triggered to generate a second pulse signal, where the first pulse signal and the second pulse signal are used to control on/off time of the transistors in the driving circuit, so as to accurately change a direction and a magnitude of current in the driving circuit.

The first pulse signal outputs a first stimulation current through the driving circuit, the second pulse signal outputs a second stimulation current through the driving circuit, and the first stimulation current is opposite to the second stimulation current; and The stimulation electrode is connected to the output end of the driving circuit, and the first stimulation current and the second stimulation current, through the stimulation electrode, act on specified positions of the human body to achieve the desired electrical stimulation effect.

**In** this embodiment, the stimulation electrode can be pre-connected to a plurality of specified stimulation positions of the human body, and the stimulation positions involve three nerves, i.e., the radial nerve, the median nerve, and the ulnar nerve respectively. A stimulation mode of the stimulation electrode is set as a random mode, that is, in each cycle (one cycle refers to a process of completing the output of the first stimulation current and the second stimulation current), the state that the stimulation electrode corresponding to one nerve outputs the stimulation pulse is switched to the state that the stimulation electrode corresponding to another nerve outputs the stimulation pulse, where this switching is known as stimulation position switching. Before the stimulation position switching, the stimulation electrode corresponding to the current nerve is controlled to output the specified stimulation pulse, and the order of the stimulation position switching is random.

Further, corresponding stimulation parameters for single stimulation pulse also vary randomly within a specified range, so that the stimulation current outputted also has randomness. Such randomness can help to prevent the neural system of a user from adapting to the stimulation mode and negating the therapeutic effect.

The first stimulation current and the second stimulation current are opposite in direction but are equal in magnitude. In a random mode, the corresponding stimulation current is outputted to any nerve position. It can be understood that when there are four electrodes A, B, C and D, the first stimulation current can be outputted from A to B, and the second stimulation current can be outputted from B to A; and the first stimulation current can also be outputted from A to C, and the second stimulation current can be outputted from C to A, and so on.

The stimulation parameters include a current amplitude, a pulse interval/stimulation frequency, a pulse width, etc. Each stimulation parameter may have a fixed value or a random value. The random current amplitude is subject to uniform distribution by a mean value within a specified range, and the mean value determines an average dose of charge on a patient's body. The range can be adjusted, but an upper limit thereof always remains below a maximum tolerance threshold. For example, the amplitude may vary between 3.5 mA and 4.5 mA, with the mean value of 4.0 mA. The dose of charge provided in this mode is equivalent to that in a fixed 4.0 mA mode.

A random pulse interval is subject to uniform distribution within a specified range and has a mean value, and the mean value determines the average dose of charge delivered. For example, the pulse interval may vary between 2 ms and 18 ms, with the mean value of 10 ms. The dose provided in this mode is equivalent to that in a fixed 100 Hz mode.

A random pulse width is subject to uniform distribution within a specified range and has a mean value, and the mean value affects the duration of electrical stimulation on the patient's body. For example, the pulse width may vary between 250 us and 350 us, with the mean value of 300 us.

The stimulation positions of the stimulation electrode involve three nerves, i.e., the radial nerve, the median nerve, and the ulnar nerve respectively, which can be switched. In each cycle, the device, before the stimulation position switching, controls the stimulation electrode corresponding to the current nerve to output a series of stimulation pulses. The order of stimulation position switching is random. Such randomness can help to prevent the neural system of a user from adapting to the stimulation mode and negating the therapeutic effect. The number of pulses in each cycle is subject to uniform distribution within a specified range and has a mean value. The mean value determines a switching frequency and an amount of electric charge allocated to the stimulation electrode corresponding to the specific nerve. For example, the stimulation electrode corresponding to the radial nerve can be controlled to output 5-15 pulses (10 pulses on average), the stimulation electrode corresponding to the median nerve can be controlled to output 5-15 pulses (10 pulses on average), and the stimulation electrode corresponding to the ulnar nerve can be controlled to output 2-8 pulses (5 pulses on average). In this mode, the electric charge is allocated to the stimulation electrode corresponding to the radial nerve, the stimulation electrode corresponding to the median nerve, and the stimulation electrode corresponding to the ulnar nerve by 40%, 40% and 20%, respectively. Assuming a mean pulse interval of 10 ms, the equivalent switching frequency is 4 Hz (250 ms).

A pseudo-random number generator can be used to create a random mode, initial random seeds can be shuffled when the device is powered on, and a resulting pseudo-random mode is imperceptible to the human body.

The driving circuit in this embodiment is an H-bridge circuit, including four transistors, the four transistors are combined to form an H-bridge circuit, and a load of the H-bridge circuit is connected to a positive pole and a negative pole of the stimulation electrode. Specifically, the H-bridge circuit includes a first n-type metal-oxide-semiconductor (NMOS) transistor, a second NMOS transistor, a first p-type metal-oxide-semiconductor (PMOS) transistor, and a second PMOS transistor, where a source electrode of the first PMOS transistor is connected to a source electrode of the second PMOS transistor and connected to a power supply, a gate electrode of the first PMOS transistor and a gate electrode of the second PMOS transistor are both connected to the same voltage/level converter, and a drain electrode of the first PMOS transistor and a drain electrode of the second PMOS transistor are connected to a positive pole and a negative pole of the stimulation electrode, respectively; the drain electrode of the first PMOS transistor is connected to the drain electrode of the first NMOS transistor, the drain electrode of the second PMOS transistor is connected to the drain electrode of the second NMOS transistor, the gate electrode of the first NMOS transistor and the gate electrode of the second NMOS transistor are connected to another voltage/level converter, and the source electrode of the first NMOS transistor and the source electrode of the second NMOS transistor are connected and grounded. The H-bridge circuit allows for voltage inversion/current reversal of the connected load.

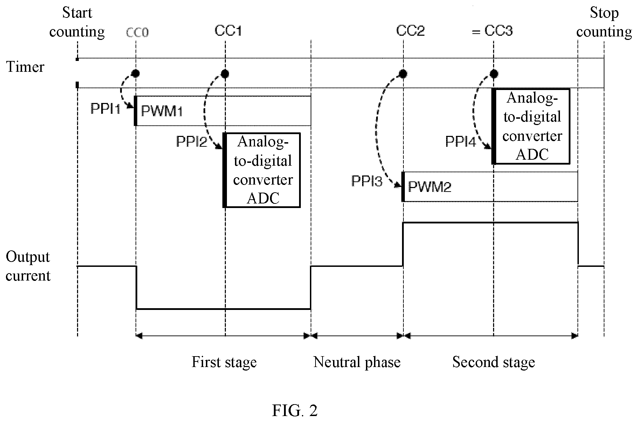

As shown in FIG. 2, the stimulation electrode in this embodiment outputs the first stimulation current and the second stimulation current, which corresponds to three stages of the stimulation pulse in a cycle, including a first stage, a neutral phase, and a second stage, where the first stage begins when the first trigger time CC0 is up after the timer starts timing, while the second stage begins when the second trigger time CC2 is up. Specifically, when CC0 - CC3 trigger time of the timer is up:
+ (CC0): the first stage begins: the timer triggers a pulse width modulation 1 (PWM1) task through the PPI. The PWM1 controls 4 transistors to generate a first phase. The pulse width is controlled by the PWM, and a clock accuracy is 62.5 ns at 16 MHz.
+ (CC1) a midpoint of the first phase: the timer triggers an analog-to-digital converter (ADC) to sample the output current of the first phase.
+ (CC2): the second stage begins: the timer triggers a PWM2 task through the PPI. The PWM2 controls 4 transistors to generate a second phase. The pulse width is controlled by the PWM, and a clock accuracy is 62.5 ns at 16 MHz.
+ (CC3) a midpoint of the second phase: the timer triggers an analog-to-digital converter (ADC) to sample the output current of the second phase.

The ADC is connected to the timer through the PPI module, and configured for acquiring an intermediate voltage of the first stimulation current when first acquisition time CC1 set by the timer is up, and acquiring an intermediate voltage of the second stimulation current when second acquisition time CC3 set by the timer is up. The ADC samples the voltage between two pulses and converts a reading to a current using the following formula: IOUT = VOUT / RREF. When the difference between the measured output current and the required current is greater than a fault threshold, a fault is detected.

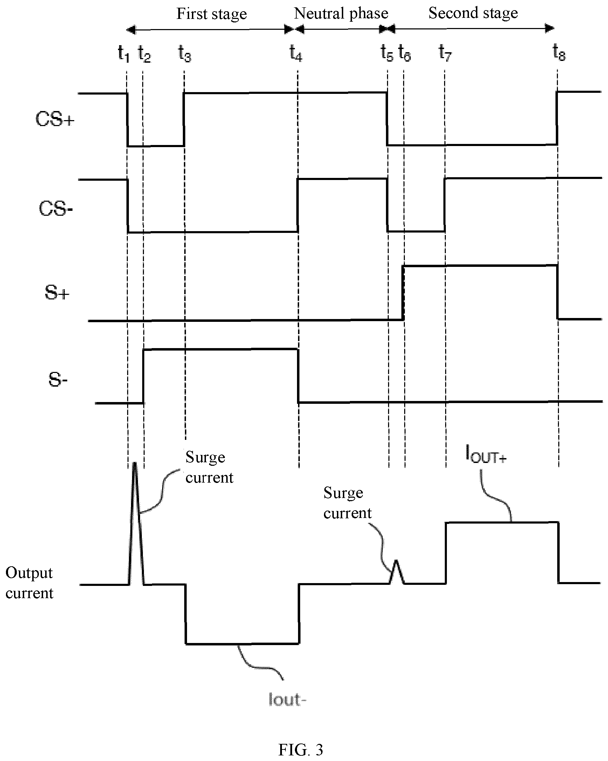

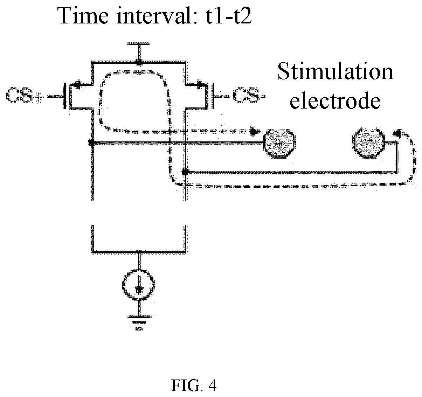

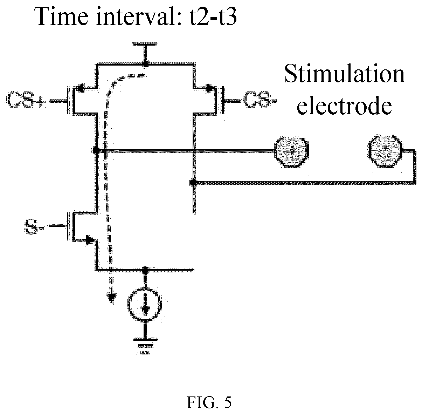

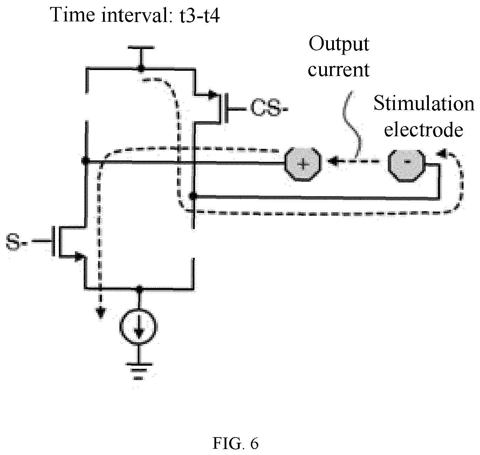

In this embodiment, in order to solve the problem of current overshoot during the activation period of the stimulation pulse, all four transistors in the H-bridge circuit at each stage of stimulation pulse generation are controlled in a coordinated manner, which is different from the traditional approach of using only two transistors. With reference to FIGS. 3-6, a specific control method is as follows:
t1 in the first stage: Both two PMOS transistors are turned on, that is, the first PMOS transistor and the second PMOS transistor are both in the "on" state, to neutralize any voltage difference between two electrodes. In this step, a very short surge current is generated (< 100 ns).
t2 in the first stage: Based on the control at the t1, one NMOS transistor is turned on to initiate the stimulation current. That is, after the first PMOS transistor and the second PMOS transistor are turned on, the first NMOS transistor is turned on, and the stimulation current does not flow through the electrodes. Instead, the stimulation current directly flows to the constant current driver.
t3 in the first stage: Based on the control at the t2, approximately 200 ns later after the first NMOS transistor is turned on, the first PMOS transistor is turned off, to redirect the stimulation current to the stimulation electrode. This marks the start of the first-stage stimulation. This strategy minimizes unnecessary peaks in the output current on the electrode.
t4 in the first stage: All PMOS and NMOS transistors are turned off, to end the first-stage stimulation.

With reference to FIG. 3, corresponding control procedures are implemented at t5, t6, t7 and t8 to generate the second-stage stimulation.

The above t1-t4 corresponds to the first stage of the stimulation pulse, t4-t5 corresponds to the neutral phase, and t5-t8 corresponds to the second stage of the stimulation pulse.

This embodiment has the following beneficial effects:
High-precision timing: Timing accuracy with the pulse widths less than 1 us is achieved in the measurement of two phases, the neutral phase and the current, which is crucial for delivering precise charge doses and ensuring a charge balance between the two phases of the biphasic stimulation pulse.
Robust operation: Timing accuracy is not affected by the load on the microcontroller. Once initiated, all modules will be triggered due to a seamless domino effect without need to rely on continuous monitoring by the main microcontroller.
Real-time current monitoring: Actual output currents are easily measured at a precise time interval in the pulse generation process, so that errors in stimulation delivery can be detected, such as electrode disconnection or electrode-skin interface damage, thereby improving safety and effectiveness.
Elimination of current overshoot: The novel switching approach effectively eliminates current surges without need of additional hardware, thereby minimizing complexity.

### Embodiment 2

In this embodiment, there is provided an electrical stimulation method that can be applied to the above electrical stimulation circuit, and the method includes the following steps:
S1: a stimulation demand is acquired, and a timer is activated according to the stimulation demand, where
   the stimulation demand can be initiated by a user who needs a stimulation electrode for electrical stimulation, and the timer starts timing after being activated;
S2: a first stimulation current is generated by a microcontroller when first trigger time set by the timer is up;
S3: a second stimulation current is generated by the microcontroller when second trigger time set by the timer is up, where the first stimulation current is opposite to the second stimulation current; and

The generation of the first stimulation current and the second stimulation current has randomness, specifically including:
each pulse parameter is randomly set based on a preset specified range, where the pulse parameters include a current amplitude, a pulse interval and a pulse width; and
the first stimulation current and the second stimulation current are generated based on the pulse parameters.

S4: the stimulation position is randomly switched, so that the stimulation electrode outputs the first stimulation current and the second stimulation current to the stimulation positions. Randomness/randomization is incorporated into a stimulation mode while still delivering the desired charge dose, which can help to prevent the neural system of a user from adapting to the stimulation mode and negating the therapeutic effect.

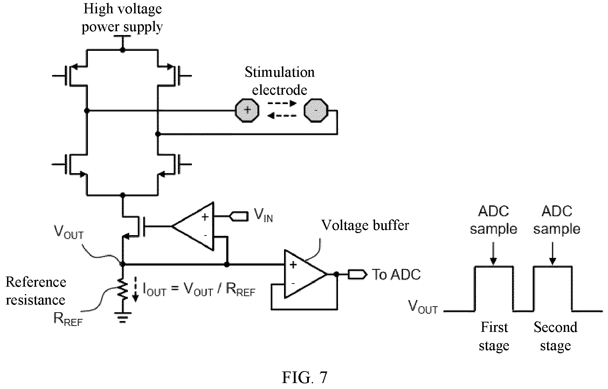

In this embodiment, in order to monitor the actual output current, as shown in FIG. 7, an intermediate voltage of the first stimulation current and the intermediate voltage of the second stimulation current are acquired. Current calculation is performed based on the intermediate voltage of the first stimulation current and the intermediate voltage of the second stimulation current according to the formula IOUT = VOUT / RREF, to obtain a first-stage stimulation current and a second-stage stimulation current.

The first-stage stimulation current and the second-stage stimulation current are compared with a preset target current, and a fault signal for triggering a fault event is generated when a difference between the first-stage stimulation current and the target current is greater than a fault threshold, and/or the difference between the second-stage stimulation current and the target current is greater than the fault threshold.

The fault threshold is proportional to the desired output current. For example, the fault threshold of a 0.3 mA output current is set as +/- 0.1 mA, while the fault threshold of a 10 mA output current is set as +/- 0.2 mA.

In this embodiment, the actual output current of each stimulation pulse is measured by calculating the voltages at both ends of a reference resistor RREF in the pulse generation process, measurements are made at the first-stage midpoint and the second-stage midpoint described above, and the results are averaged. The goal is to continuously monitor the real-time current output to respond promptly to potential faults. Such faults often occur when an electrode-skin interface is damaged, thereby hindering a stimulator's capability to provide the expected stimulation current. This mechanism can protect the users from potential electric shocks in the event of faults.

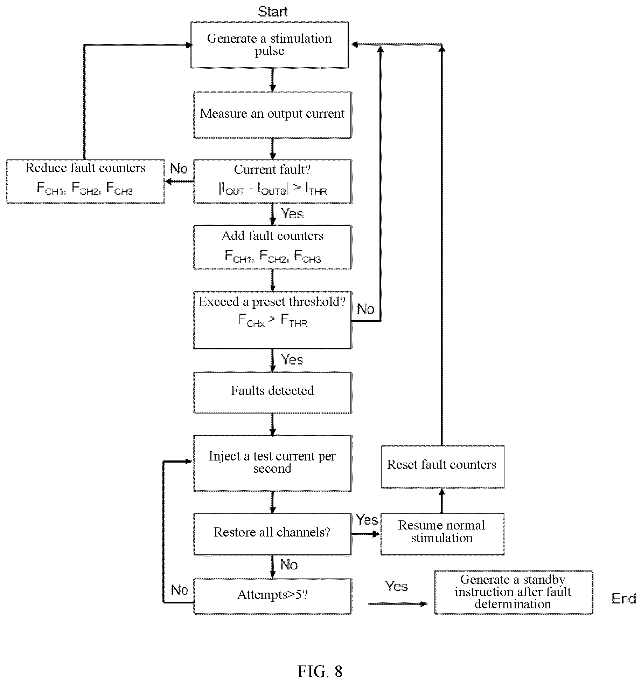

To enhance fault detection robustness and prevent false alarms, as shown in FIG. 8, the electrical stimulation method further includes:
an error counter is configured for each channel in the electrical stimulation circuit, an increment signal is generated and sent to a counter when a fault event occurs, so that the counter counts up; a decrement signal is generated and sent to the counter when no fault event occurs, so that the counter counts down; and
a standby instruction is generated when a count value of the counter exceeds a preset threshold, where the standby instruction is used to control the stimulation electrode to stop outputting the stimulation current. When a fault occurs, the counter counts up, and otherwise, the counter counts down; when the value of the counter exceeds the specified preset threshold, a fault is detected. For example, when the preset threshold of faults is 10, approximately 100 ms and 10 pulses are needed to detect the faults.

A fault detected is designated to be pending; and the stimulation circuit executes the standby instruction to immediately stop the electrical stimulation so as to prevent potential harm to the user. However, recovery attempts are made by generating a test pulse of a preset amplitude (such as a 0.3 mA amplitude) once per second, to evaluate the channel connection separately. When determining that the output current of the stimulation electrode matches the test pulse, it indicates that the channel under test has been recovered, and when there is a significant difference between the output current of the stimulation electrode and the test pulse, it indicates that the channel under test has not been recovered yet.

When all channels have been successfully recovered, normal stimulation is resumed, any parameter updates made during the recovery period are retained, and the stimulation electrode re-outputs the first stimulation current and the second stimulation current. However, after five failed attempts, the fault is designated as having been confirmed. Then the device terminates a stimulation session and notifies the user through an LED indicator and an application.

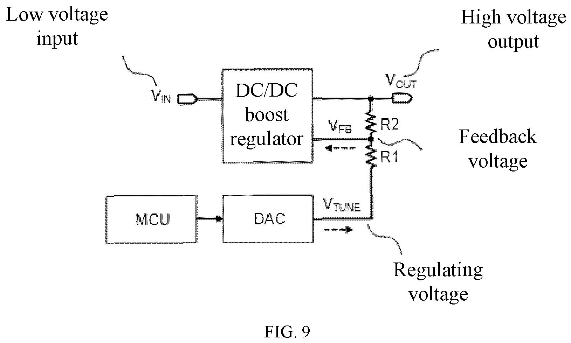

In some embodiments, a power supply voltage can also be adjusted according to the required output current. When the stimulator only needs to generate low output current, the voltage of a high voltage power supply can decrease; and similarly, when the stimulator needs to generate high output current, the voltage of the high-voltage power supply can increase accordingly, thereby achieving the effect of an adaptive high-voltage power supply. Specific details are as follows:
A DC/DC boost regulator is added for the stimulation circuit, and a digital-to-analog converter (DAC) is added for a feedback branch of the DC/DC boost regulator to modify a high-voltage generator circuit, as shown in FIG. 9. The DAC is controlled by a microcontroller unit (MCU). Through adjusting of VTUNE, a value of VOUT can be adjusted according to the following formula: VOUT=VTUNE+(VFB - VTUNE) (R1+R2)/R1.

The VFB is a fixed feedback voltage specified by the DC/DC boost regulator, and R1 and R2 are both reference resistances.

When VTUNE=0, VOUT=VFB (R1+R2)/R1, which is the maximum value that can be generated by the electrical stimulation circuit. When VTUNE=VFB, VOUT=VTUNE, which is the minimum value.

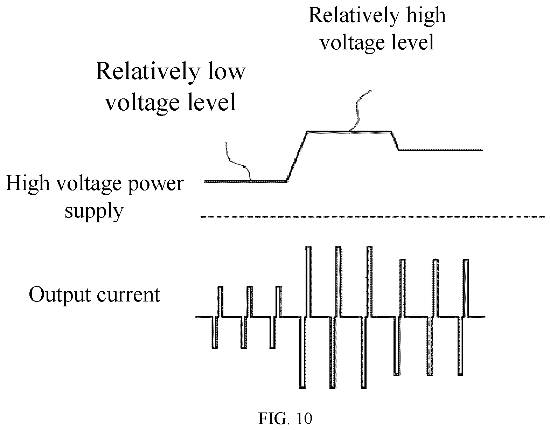

As shown in FIG. 10, the voltage of the high-voltage power supply can increase or decrease in real time according to the value of the output current.

The above adaptive scheme has two advantages: firstly, power consumption can be significantly reduced by adjusting the voltage of the high-voltage power supply as needed; although the electrical stimulation circuit is designed to generate a maximum of 10 mA at 100 V, the user does not need the maximum stimulation current in most cases; and when the user only needs the current of 5 mA, the voltage of the high-voltage power supply can be reduced to 80 V, to significantly reduce power consumption and extend battery life.

Secondly, reducing the voltage of the high-voltage power supply helps to capture current faults more easily; and when an electrode interface is slightly damaged, current faults can be detected more easily by reducing the voltage of the high-voltage power supply.

For circuit modules and functions corresponding to the method in this embodiment of the present invention, please refer to the corresponding description of the above electrical stimulation circuit, which will not be repeated herein.

### Embodiment 3

In an embodiment of the present invention, there is provided an electronic device, and the electronic device includes a memory and a processor, where the memory has stored thereon a computer program executable by a processor. When the processor executes this computer program, the electrical stimulation method in the above embodiment is implemented. One or more memories and processors can be configured.

The electronic device further includes:
a communication interface configured for communication with external devices and data exchange.

When the memory, the processor and the communication interface are implemented independently, they can be interconnected and communicate with each other through a bus. The bus can be an industry standard architecture (ISA) bus, a peripheral component interconnect (PCI) bus, or an extended industry standard architecture (EISA) bus, etc. The bus can be divided into an address bus, a data bus, a control bus, etc.

Optionally, in terms of specific implementation, when the memory, the processor and the communication interface are integrated on a single chip, they can communicate with each other through an internal interface.

In an embodiment of the present invention, there is provided a computer-readable storage medium on which a computer program is stored, and when the computer program is executed by the processor, the method provided in the embodiment of the present invention is implemented.

In an embodiment of the present invention, there is further provided a chip, and the chip includes a processor configured for calling and executing the instructions stored in the memory, so that a communication device provided with the chip can implement the method provided in the embodiment of the present invention.

In an embodiment of the present invention, there is further provided a chip, and the chip includes an input interface, an output interface, a processor, and a memory, where the input interface, the output interface, the processor and the memory are interconnected through an internal connection pathway, the processor is configured for executing a computer code stored in the memory, and when the code is executed, the processor executes the method in the embodiment of the present invention.

It should be understood that the processor can be a central processing unit (CPU), a microprocessor unit (MPU), any other general-purpose processor, a digital signal processor (DSP), an application specific integrated circuit (ASIC), a field-programmable gate array (FPGA), or any other programmable logic device (PLD), a discrete gate or a transistor logic device, or a discrete hardware component, etc. The general-purpose processor can be a microprocessor, or any conventional processor. It is worth noting that the processor can be a processor compatible with an advanced reduced instruction set computer (RISC) machine (ARM) architecture.

Further, optionally, the above memory may include a read-only memory (ROM), a random access memory (RAM), or a non-volatile random access memory (NVRAM). The memory can be either a volatile memory or a non-volatile memory, or may include both the volatile memory and the non-volatile memory. The non-volatile memory may include the ROM, a programmable ROM (PROM), an erasable ROM (EPROM), an electrically EPROM (EEPROM) or a flash memory. The volatile memory may include an RAM used for an external cache memory. By means of illustrative and non-limiting description, many forms of RAM are available, such as a static RAM (SRAM), a dynamic RAM (DRAM), a synchronous DRAM (SDRAM), a double data rate SDRAM (DDR SDRAM), an enhanced SDRAM (ESDRAM), a synchlink DRAM (SLDRAM), and a direct rambus RAM (DR RAM).

In the above embodiments, full or partial implementation is realized through software, hardware, firmware, or any combination thereof. When software is used for implementation, full or partial implementation can be realized in the form of a computer program product. The computer program product includes one or more computer instructions. When loading and executing a computer program instruction on a computer, all or part of processes or functions according to the present invention are generated. The computer can be a general-purpose computer, a dedicated computer, a computer network, or any other programmable device. The computer instructions can be stored in a computer-readable storage medium or transmitted from one computer-readable storage medium to another computer-readable storage medium.

**In** the description of the present specification, the description of reference terms such as "one embodiment", "some embodiments", "example", "specific example" or "some examples" means that specific features, structures, materials or characteristics described in combination with the embodiment or example are included in at least one embodiment or example of the present invention. Moreover, the specific features, structures, materials or characteristics described may be combined in a suitable manner in any one or more embodiments or examples. Further, those skilled in the art may integrate and combine different embodiments or examples described in the present specification, as well as the features of different embodiments or examples without contradiction.

Furthermore, the terms "first" and "second" are merely for the purpose of description, and cannot be construed as indicating or implying relative importance or implicitly specifying the number of technical features indicated. Thus, a feature defined with "first" and "second" may explicitly or implicitly include at least one of the feature. In the description of the present invention, "a plurality of" means two or more, unless expressly specified otherwise.

The foregoing descriptions are merely specific implementations of the present invention, and are not intended to limit the protection scope of the present invention. Various substitutions or changes made by a person skilled in the art easily within the technical scope disclosed in the present invention shall fall within the protection scope of the present invention. Therefore, the protection scope of the present invention should be subject to a protection scope of the claims.

## Claims

1. An electrical stimulation circuit, **characterized by** comprising:
a timer, being configured for timing;
a pulse width modulator, being connected to the timer, wherein a first pulse signal is generated when first trigger time set by the timer is up, and a second pulse signal is generated when second trigger time set by the timer is up;
a driving circuit, being connected to the pulse width modulator, wherein the first pulse signal outputs a first stimulation current through the driving circuit, the second pulse signal outputs a second stimulation current through the driving circuit, and the first stimulation current is opposite to the second stimulation current; and
a stimulation electrode, being connected to the driving circuit, wherein the stimulation electrode is in contact with a plurality of stimulation positions, and configured for randomly outputting the first stimulation current and the second stimulation current to any stimulation position.

2. The electrical stimulation circuit according to claim 1, **characterized by** further comprising:
a peripheral interconnection module, wherein the timer is connected to the pulse width modulator through the peripheral interconnection module; and
an analog-to-digital converter, being connected to the timer through the peripheral interconnection module, and being configured for acquiring an intermediate voltage of the first stimulation current when first acquisition time set by the timer is up, and acquiring an intermediate voltage of the second stimulation current when second acquisition time set by the timer is up, wherein the intermediate voltage of the first stimulation current and the intermediate voltage of the second stimulation current are used for fault detection.

3. The electrical stimulation circuit according to claim 2, **characterized by** further comprising:
a voltage/level converter, being configured for performing voltage/level conversion of the first pulse signal and the second pulse signal and outputting to the driving circuit, wherein an input end thereof is connected to the pulse width modulator, and an output end thereof is connected to the driving circuit; and
the driving circuit comprises four transistors, the four transistors are combined to form an H-bridge circuit, and a load of the H-bridge circuit is connected to the stimulation electrode.

4. The electrical stimulation circuit according to claim 3, wherein the H-bridge circuit comprises a first n-type metal-oxide-semiconductor (NMOS) transistor, a second NMOS transistor, a first p-type metal-oxide-semiconductor (PMOS) transistor, and a second PMOS transistor, wherein a source electrode of the first PMOS transistor is connected to a source electrode of the second PMOS transistor and connected to a power supply, a gate electrode of the first PMOS transistor and a gate electrode of the second PMOS transistor are both connected to the same voltage/level converter, and a drain electrode of the first PMOS transistor and a drain electrode of the second PMOS transistor are connected to a positive pole and a negative pole of the stimulation electrode, respectively; the drain electrode of the first PMOS transistor is connected to the drain electrode of the first NMOS transistor, the drain electrode of the second PMOS transistor is connected to the drain electrode of the second NMOS transistor, the gate electrode of the first NMOS transistor and the gate electrode of the second NMOS transistor are connected to another voltage/level converter, and the source electrode of the first NMOS transistor and the source electrode of the second NMOS transistor are connected and grounded through a constant current driver.

5. The electrical stimulation circuit according to claim 4, **characterized in that** the first PMOS transistor and the second PMOS transistor are controlled to be in the "on" state at the first trigger time, so that the stimulation current flows to the stimulation electrodes to neutralize a voltage difference between two stimulation electrodes.

6. The electrical stimulation circuit according to claim 5, **characterized in that** when the first PMOS transistor and the second PMOS transistor are both in the "on" state, the first NMOS transistor is controlled to be in the "on" state, and the stimulation current flows through the first PMOS transistor and the first NMOS transistor to the constant current driver and then a grounding location.

7. The electrical stimulation circuit according to claim 6, **characterized in that** when the first PMOS transistor, the second PMOS transistor, and the first NMOS transistor are all in the "on" state, the first PMOS transistor is controlled to turn off, so that the stimulation current flows to the stimulation electrode.

8. The electrical stimulation circuit according to claim 1, **characterized in that** the stimulation positions comprise a corresponding position of a radial nerve, a corresponding position of a median nerve, and a corresponding position of an ulnar nerve.

9. The electrical stimulation circuit according to claim 1, **characterized by** further comprising:
a microcontroller, being equipped with the timer and the pulse width modulator; and
a DC/DC boost regulator, wherein a feedback branch thereof is connected to a digital-to-analog converter, and the digital-to-analog converter is connected to a microcontroller and configured for modifying a power supply voltage according to required output current.

10. An electrical stimulation method, **characterized by** being applied to the electrical stimulation circuit according to any one of claims 1-15, comprising the following steps:
acquiring a stimulation demand, and activating the timer according to the stimulation demand;
generating the first stimulation current when the first trigger time set by the timer is up;
generating the second stimulation current when the second trigger time set by the timer is up, wherein the first stimulation current is opposite to the second stimulation current; and
randomly switching the stimulation positions, so that the stimulation electrode outputs the first stimulation current and the second stimulation current towards the stimulation positions.

11. The electrical stimulation method according to claim 10, **characterized in that** a method of generating the first stimulation current and the second stimulation current comprises the following steps:
randomly setting pulse parameters based on a preset specified range, wherein the pulse parameters comprises a current amplitude, a pulse interval and a pulse width; and
generating the first stimulation current and the second stimulation current based on the pulse parameters.

12. The electrical stimulation method according to claim 10, **characterized by** further comprising the following steps:
acquiring the intermediate voltage of the first stimulation current and the intermediate voltage of the second stimulation current;
calculating a first-stage stimulation current and a second-stage stimulation current according to the intermediate voltage of the first stimulation current and the intermediate voltage of the second stimulation current, respectively; and
comparing the first-stage stimulation current and the second-stage stimulation current with a preset target current, and generating a fault signal for triggering a fault event when a difference between the first-stage stimulation current and the target current is greater than a fault threshold, and/or the difference between the second-stage stimulation current and the target current is greater than the fault threshold.

13. The electrical stimulation method according to claim 12, **characterized by** further comprising the following steps:
generating an increment signal and sending the increment signal to a counter when a fault event occurs, so that the counter counts up; generating a decrement signal and sending the decrement signal to the counter when no fault event occurs, so that the counter counts down; and
generating a standby instruction when a cumulative count value of the counter exceeds a preset threshold, wherein the standby instruction is used to control the stimulation electrode to stop outputting the stimulation current.

14. The electrical stimulation method according to claim 13, **characterized by** further comprising the following steps:
generating a test pulse with a preset amplitude when the standby instruction is executed; and
when determining that an output current of the stimulation electrode matches the test pulse, restoring the stimulation electrode to output the first stimulation current and the second stimulation current.

15. The electrical stimulation method according to claim 10, **characterized by** further comprising:
obtaining the required output current and adjusting the power supply voltage of the electrical stimulation circuit according to the required output current.
